# EUROPÄISCHE PATENTSCHRIFT

(11) **EP 4 355 264 B1**
(45) Veröffentlichungstag und Bekanntmachung des Hinweises auf die Patenterteilung: **08.04.2026**
(21) Anmeldenummer: 22735111.1
(22) Anmeldetag: 15.06.2022
(51) Int. Cl.: A61F 2/16, A61B 90/98, G16H 10/65, A61B 5/145, A61B 5/03, A61B 5/00

(54) **OPHTHALMISCHES IMPLANTAT UND VERFAHREN ZUR HERSTELLUNG EINES SOLCHEN**
OPHTHALMIC IMPLANT AND METHOD FOR PRODUCING SAME
IMPLANT OPHTALMIQUE ET PROCÉDÉ POUR LE PRODUIRE

(30) Priorität: 15.06.2021 DE 102021206093; 13.08.2021 DE 102021121166
(43) Veröffentlichungstag der Anmeldung: 24.04.2024
(73) Patentinhaber: Carl Zeiss Meditec AG, 07745 Jena (DE)
(72) Erfinder: SCHREIBER, Benjamin, 10589 Berlin (DE); MASCH, Jennifer-Magdalena, 10589 Berlin (DE); BADUR, Thorben, 10589 Berlin (DE); DAMM, Niklas, 10589 Berlin (DE)
(74) Vertreter: Pearl Cohen EU
(86) Internationale Anmeldenummer: PCT/EP2022/066281
(87) Internationale Veröffentlichungsnummer: WO 2022/263495

(56) Entgegenhaltungen:
- EP-A2- 2 846 182
- WO-A1-2015/081298
- US-A1- 2006 232 408
- US-A1- 2006 267 768
- US-A1- 2017 103 363

## Beschreibung

Ausführungsformen der Erfindung betreffen ein ophthalmisches Implantat mit einer kontaktlos elektronisch auslesbaren Datenspeichereinheit und ein Verfahren zur Herstellung eines ophthalmischen Implantats mit einer kontaktlos elektronisch auslesbaren Datenspeichereinheit. Die Ausführungsformen liegen somit insbesondere auf dem Gebiet der ophthalmischen Implantate und insbesondere auf dem Gebiet der Intraokularlinsen.

Medizinische Implantate und insbesondere ophthalmische Implantate, wie etwa Intraokularlinsen (IOLs), müssen typischerweise eine Rückverfolgbarkeit über die gesamte Lebensdauer des Implantats bieten. Diese Anforderung, die zum Beispiel in der europäischen Verordnung über Medizinprodukte (Medical Device Regulation) definiert ist, muss für ein Medizinprodukt erfüllt werden, um zugelassen zu werden.

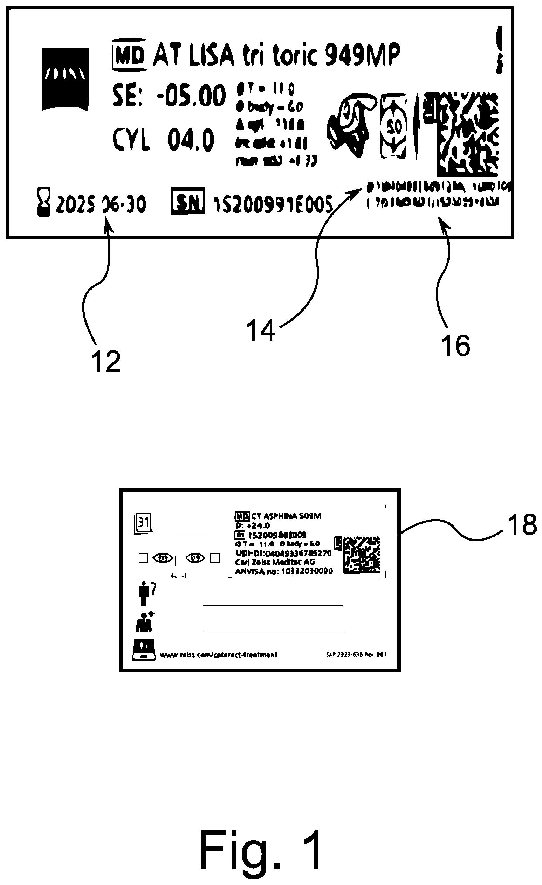

Eine anforderungsgemäße Rückverfolgbarkeit ist durch die eindeutige Produktidentifikation (engl.: Unique Device Identification, UDI) gegeben, die typischerweise eine Kombination aus der Hersteller-ID und einer Seriennummer in Form einer globalen Handelselementnummer (engl.: Global Trade Item Number, GTIN) bereitgestellt wird. Zusätzlich sind Informationen wie Chargennummer und Verfallsdatum erforderlich. Beispiele für handelsübliche Bezeichnungen zur Identifikation eines ophthalmischen Implantats sind in Figur 1 gezeigt, welche in der Figurenbeschreibung näher erläutert sind.

Für IOLs werden typischerweise zusätzliche Informationen benötigt: Diese umfassen Angaben zur Dioptrienstärke und zylindrischen Stärke, zum IOL-Material und zur Modellnummer. Um eine geeignete IOL für einen Patienten auszuwählen, werden typischerweise Berechnungskonstanten "A-Konstanten" aus einer Datenbank benötigt, die spezifisch für einen bestimmten IOL-Typ sind. Falls der Patient beispielsweise eine astigmatismuskorrigierende IOL benötigt, beschreibt die Implantationsachse den Drehwinkel des Implantats im Auge.

Da viele ophthalmische Implantate und insbesondere IOLs eine faltbare Struktur aufweisen, welche eine Implantation im zusammengefalteten Zustand mit anschließender Entfaltung ermöglichen, sind herkömmlicherweise Informationen zur Identifikation und zur Bestimmung weiterer Eigenschaften nicht direkt mit dem ophthalmischen Implantat verbunden bzw. daran angebracht. Insbesondere im Fall einer IOL, welche hauptsächlich eine klare Linse aufweist, sind UDI- und IOL-Informationen oft nicht mit dem Implantat verbunden.

Im Stand der Technik sind IOLs des Herstellers OPHTECH GmbH bekannt, welche einen Micro-QR Code mit produktspezifische Informationen in der Haptik, z. B. eine UDI und andere Implantationsinformationen, aufweisen (https://www.ophtec.com/company/news?news id=947587). Um die optische Wirkung der IOL nicht zu beeinträchtigen ist der Micro-QR Code so auf der IOL platziert, dass dieser Bereich im implantierten Zustand hinter der Iris verborgen liegt. Dies führt dazu, dass dieser im implantierten Zustand nicht auslesbar ist. Zusätzlich ist herkömmlicherweise ein Auslesen ebenfalls nicht möglich, wenn die IOL in einem Applikator gefaltet ist.

Ein Code mit besserer Sichtbarkeit ist auf einer IOL schwer anzubringen, ohne die optische Leistung des Implantats zu beeinträchtigen und dennoch im implantierten Zustand leicht sichtbar zu sein. Daher werden bei IOLs herkömmlicherweise die Informationen über die Verpackung und/oder ein gesondertes Informationsblatt oder eine Karte (z. B. Patientenkarte) zur Verfügung gestellt. Diese Informationen können jedoch mit der Zeit verloren gehen, da diese nicht mit der IOL verbunden sind.

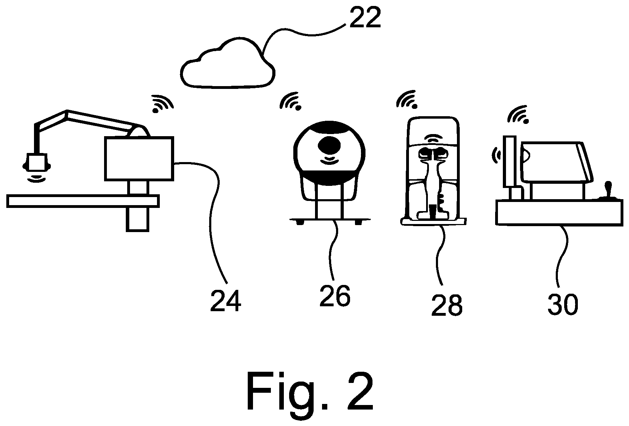

Grundsätzlich ist eine eindeutige und unverwechselbare Rückverfolgbarkeit des Implantats von der Produktion, über die Logistik, während des Implantationsvorgangs, im implantierten Zustand und ggf. nach Explantation für das Reklamationsmanagement notwendig. Bereits während der Implantation und in-vivo nach der Implantation gibt es viele verschiedene Anlässe, bei denen eine Verifizierung des Implantats und damit eine Rückverfolgbarkeit hilfreich ist, wie in Figur 2 dargestellt.

Die WO 2012/157623 A1 beschreibt einen aktiven Sensor, dessen Chip zum Erfassen eines Augendruckes in der Haptik angeordnet ist, wobei im Randbereich des IOL-Optikkörpers in dessen Mittenebene Antennen-Windungen zur Übertragung der gemessenen Werte aus dem Auge heraus eingebracht sind.

In der EP2620802 A1 ist eine Kontaktlinse dargestellt, die eine AntennenAnordnung innerhalb des Nicht-Optikteils enthält, die dazu dient, Daten zu übertragen.

In der US 2009/0244477 A1 ist eine ophthalmische Linse beschrieben, die im Nicht-Optikteil einen Energie-Rezeptor (=Antenne) enthält, der mit einer Komponente gekoppelt ist. Die Komponente kann ein RFID-Chip sein. Die Antenne ist innerhalb der Linse angeordnet.

Die WO 2015/081298 A1 beschreibt eine Intraokularlinse mit integriertem Display, welche diverse auf der Haptik angeordnete elektronische Bauteile aufweist. Gemäß der Offenbarung der D1 kann eines der Bauteile ein Sensor sein, der wiederum als RFID Chip ausgebildet sein kann.

Die US 2006/267768 A1 beschreibt ein System zur Überwachung einer Verwendungsdauer von ophthalmischen Produkten, welche primär als Kontaktlinsen ausgestaltet sind. Dabei können die Kontaktlinsen RFID Tags aufweisen, welche sodann ausgelesen werden können, um Informationen über die Kotaktlinse auszulesen.

Die EP 2 846 182 A2 beschreibt ein System mit einer ophthalmischen Linse und einem externen Gerät, welche drahtlos miteinander kommunizieren können. Die US 2017/103363 A1 beschreibt ein System zum Inventurmanagement, welches bei der Identifikation der IOL in einer chirurgischen Anwendung unterstützen kann. Die IOL kann dabei Sensoren aufweisen, welche einem Benutzer Informationen bereitstellen.

Es besteht daher die Aufgabe, ein ophthalmisches Implantat bereitzustellen, welches eine einfache Herstellbarkeit und zuverlässige Identifikation über die gesamte Lebenszeit des ophthalmischen Implantats ermöglicht, insbesondere in implantiertem Zustand.

Die Aufgabe wird gelöst durch ein ophthalmisches Implantat mit einer Datenspeichereinheit und ein Verfahren zur Herstellung eines ophthalmischen Implantats mit einer kontaktlos elektronisch auslesbaren Datenspeichereinheit mit den Merkmalen der jeweiligen unabhängigen Ansprüche. Optionale Ausgestaltungen sind in den Unteransprüchen und in der Beschreibung angegeben.

Eine erste Ausführungsform betrifft ein ophthalmisches Implantat umfassend einen optischen Teil mit einer optischen Wirkung und einen mit dem optischen Teil verbundenen oder verbindbaren nicht-optischen Teil. Ferner umfasst das ophthalmische Implantat eine im implantierten Zustand des ophthalmischen Implantats kontaktlos elektronisch auslesbare Datenspeichereinheit, welche kontaktlos elektronisch auslesbare Daten enthält, anhand welcher das ophthalmische Implantat identifizierbar ist. Dabei ist die elektronisch auslesbare Datenspeichereinheit auf den nicht-optischen und/oder den optischen Teil aufgebracht und derart angeordnet, dass die optische Wirkung des optischen Teils im Wesentlichen nicht durch die elektronisch auslesbare Datenspeichereinheit beeinträchtigt wird.

Eine weitere Ausführungsform betrifft ein Verfahren zur Herstellung eines ophthalmischen Implantats. Das Verfahren umfasst ein Bereitstellen eines ophthalmischen Implantats mit einem optischen Teil und einem mit dem optischen Teil verbundenen oder verbindbaren nicht-optischen Teil, sowie ein Aufbringen einer kontaktlos elektronisch auslesbaren Datenspeichereinheit derart, dass die optische Wirkung des optischen Teils im Wesentlichen nicht durch die elektronisch auslesbare Datenspeichereinheit beeinträchtigt wird.

Ein ophthalmisches Implantat ist dabei ein Implantat, welches zur Implantation in ein Auge eines Patienten, insbesondere also ein menschliches oder tierisches Auge, geeignet ist. Ein ophthalmisches Implantat kann optional als eine Intraokularlinse (IOL) ausgebildet sein. Optional ist demnach das ophthalmische Implantat als Intraokularlinse ausgebildet, wobei der optische Teil eine Linse mit einer refraktiven und/oder diffraktiven Wirkung aufweisen kann oder als solche ausgebildet sein kann. Alternativ oder zusätzlich kann das ophthalmische Implantat einen Sensor aufweisen, um einen oder mehrere Parameter im Inneren des Auges zu bestimmen, wie etwa den Innendruck des Auges und/oder eine Konzentration einer Substanz, wie etwa von Zucker.

Der optische Teil mit einer optischen Wirkung des ophthalmischen Implantats kann beispielsweise ein solcher Teil des ophthalmischen Implantats sein, welcher eine refraktive und/oder diffraktive Wirkung aufweist. Insbesondere kann der optische Teile als eine optische Linse ausgebildet sein oder eine solche umfassen. Dabei kann es essentiell sein, dass der optische Teil optisch transparent ist und hindurchtretendes Licht möglichst nicht durch unerwünschte Einflüsse abgelenkt und/oder reflektiert und/oder absorbiert wird.

Der nicht-optische Teil kann dabei ein weiterer Teil des ophthalmischen Implantats sein, welcher keine optische Wirkung aufweist. Insbesondere kann der nicht-optische Teil ein solcher Teil sein, welcher nicht an einer optischen Abbildung mitwirkt. Der nicht-optische Teil kann, insbesondere im Fall einer IOL, eine oder mehrere Haptiken aufweisen oder daraus bestehen, welche zur Stabilisierung und/oder Befestigung des ophthalmischen Implantats im Auge, beispielsweise im Kapselsack, dienen. Auch kann der nicht-optische Teil dazu dienen, eine Kraft auf den optischen Teil zu übertragen, um etwa eine refraktive Wirkung des optischen Teils zu ändern. Beispielsweise kann das Auge mittels des Ziliarmuskels eine Kraft auf den nicht-optischen Teil ausüben, welche sodann durch den nicht-optischen Teil auf den optischen Teil übertragen wird und/oder in eine Verformung des optischen Teils überführt wird. Dass der nicht-optische Teil mit dem optischen Teil verbunden ist, bedeutet dabei, dass ein mechanischer Kontakt zwischen dem optischen und nicht-optischen Teil besteht. Optional können der optische und der nicht-optische Teil zusammen einstückig ausgebildet sein und das ophthalmische Implantat bilden. Dass alternativ der nicht-optische Teil mit dem optischen Teil verbindbar ist, bedeutet dabei, dass der optische mit dem nicht-optischen Teil zusammengesetzt werden kann, sodass diese nach dem Zusammensetzen miteinander verbunden sind. Manche Ausführunsformen können derart ausgestaltet sein, dass ein Zusammensetzen des ophthalmsichen Implantats ein Verbinden des optischen und nicht-optischen Teils während des Implantationsvorgangs umfasst. Beispielsweise kann ein ophthalmisches Implantat als eine modulare und/oder mehrteilige IOL vorliegen, welche erst im Kapselsack zusammengesetzt wird, was ein Verbinden des optischen und nicht-optischen Teils umfasst.

Dass die Datenspeichereinheit kontaktlos elektronisch auslesbar ist, bedeutet dabei, dass kein mechanischer und elektrischer Kontakt zwischen einem Lesegerät und der Datenspeichereinheit hergestellt werden muss, um diese auszulesen. Mit anderen Worten ist keine Kabelverbindung zwischen einem Lesegerät erforderlich, um die Daten aus der Datenspeichereinheit elektronisch auszulesen. Der Begriff "kontaktlos" betrifft dabei einen nicht erforderlichen Kontakt zwischen der Datenspeichereinheit und einem Lesegerät. Gemäß manchen optionalen Ausführungsformen kann es jedoch erforderlich sein, dass ein Kontakt zwischen Gewebe des Auges, beispielsweise der Hornhaut, und dem Lesegerät hergestellt wird, wobei dabei dennoch kein Kontakt zwischen der Datenspeichereinheit und dem Lesegerät besteht. Gemäß anderen Ausführungsformen ist jedoch auch kein Kontakt zwischen Gewebe des Auges und dem Lesegerät erforderlich, um mittels des Lesegeräts Daten aus der Datenspeichereinheit kontaktlos auszulesen. Gemäß einer optionalen Ausführunsform kann das Lesegerät dazu eingerichtet sein, einen mechanischen Kontakt mit der Schläfe des Patienten während des Auslesens der Daten aus der Datenspeichereinheit herzustellen. Dass das Auslesen der Daten elektronisch erfolgt, bedeutet dabei, dass die Daten in elektronischer Form, beispielsweise in Bits und/oder Bytes, vorliegen und von einer Recheneinheit gelesen werden können. Beispielsweise kann das kontaktlose elektronische Auslesen mittels einer Funkverbindung zwischen dem Lesegerät und der Datenspeichereinheit erfolgen. Die Funkverbindung kann dabei mittels elektromagnetischer Wellen bereitgestellt werden, welche optional das Gewebe des Auges zumindest teilweise durchdringen können.

Dass die Datenspeichereinheit bzw. die Daten in einem implantierten Zustand des ophthalmischen Implantats auslesbar sind, schließt selbstverständlich nicht aus, dass die Datenspeichereinheit bzw. die Daten auch in einem nicht-implantierten Zustand des ophthalmischen Implantats kontaktlos auslesbar sind. Vielmehr ist die Datenspeichereinheit optional auch vor dem Implantationsvorgang kontaktlos auslesbar. Optional kann die Datenspeichereinheit auch dann kontaktlos auslesbar sein, wenn diese von einer Verpackung umschlossen ist. Dies bietet den Vorteil, dass das ophthalmische Implantant zur Identifikation nicht aus der Verpackung entnommen werden muss.

Dass die optische Wirkung des optischen Teils im Wesentlichen nicht durch die elektronisch auslesbare Datenspeichereinheit beeinträchtigt wird bedeutet dabei, dass die auf dem ophthalmischen Implantat aufgebrachte Datenspeichereinheit die optische Wirkung des optischen Teils nicht oder nur in solch geringem Umfang verschlechtert, dass die verbleibende optische Wirkung zumindest ausreichend für die beabsichtigte Funktion des ophthalmischen Implantats ist.

Die Ausführungsformen bieten den Vorteil, dass eine zuverlässige und einfache Identifikation des ophthalmischen Implantats ermöglicht wird, ohne dass dazu separate Informationsträger, welche nicht mit dem ophthalmischen Implantat verbunden sind, benötigt werden. Insbesondere erlauben die Ausführungsformen auch dann eine einfache und zuverlässige Identifikation, wenn das ophthalmische Implantat bereits im Auge implantiert ist. Dadurch, dass das Auslesen der Daten kontaktlos und elektronisch funktioniert, kann insbesondere auch dann ein Auslesen und entsprechend eine Identifikation des ophthalmischen Implantats erfolgen, wenn die Datenspeichereinheit teilweise oder vollständig in implantiertem Zustand des ophthalmischen Implantats verdeckt ist, beispielsweise durch die Iris des Auges. Dies bietet wiederum den Vorteil, dass die Datenspeichereinheit bewusst an solchen Stellen und Teilen des Implantats angeordnet werden kann, welche nach der Implantation nicht von außen sichtbar und zugänglich sind.

Außerdem bietet die Erfindung den Vorteil, dass durch die Aufbringung der Datenspeichereinheit auf den optischen Teil und/oder den nicht-optischen Teil die Anordnung aus optischem und nicht-optischem Teil zunächst ohne die Datenspeichereinheit hergestellt werden kann. Im Gegensatz zu herkömmlichen ophthalmischen Implantaten, bei welchen eine Antennenstruktur beispielsweise in eine IOL integriert (z.B. eingegossen) ist, bietet dies den Vorteil, dass Komplikationen, wie etwa Verspannungen und unerwünschte Temperaturunterschiede, bei der Herstellung des optischen und nicht-optischen Teils vermieden werden können. Auch in Fällen, in denen eine mechanische Bearbeitung des optischen und/oder nicht-optischen Teils zur Herstellung des ophthalmischen Implantats erforderlich ist, bietet die Möglichkeit des nachträglichen Aufbringens der Datenspeichereinheit den Vorteil, dass eine etwaige Beschädigung der Datenspeichereinheit durch die mechanische Bearbeitung vermieden werden kann, da diese nach Abschluss der mechanischen Bearbeitung aufgebracht werden kann. Zudem bietet dies den Vorteil, dass der optische und nicht-optische Teil vor Aufbringen der Datenspeichereinheit nur auf etwaige Fehler überprüft werden kann und dadurch die Aufbringung einer Datenspeichereinheit auf ein fehlerbehaftetes ophthalmisches Implantat vermieden werden kann. Somit können der Ausschuss und entsprechend die Herstellungskosten reduziert werden.

Auch bieten die Ausführungsformen den Vorteil, dass im Gegensatz zu optischen Methoden zum Auslesen von Daten keine Sichtverbindung durch die Verwendung eines kontaktlosen elektronischen Auslesens zwischen dem Lesegerät und der Datenspeichereinheit zwingend erforderlich ist.

Dass die Datenspeichereinheit auf den optischen und/oder nicht-optischen Teil des ophthalmischen Implantats aufgebracht ist, bedeutet dabei, dass die Datenspeichereinheit zumindest teilweise und optional vollständig auf einer Oberfläche des optischen und/oder nicht-optischen Teils angeordnet und daran befestigt ist. Optional können im optischen und/oder nicht optischen Teil Strukturen zur Aufbringung der Datenspeichereinheit oder von Teilen davon bereitgestellt werden. Optional können eine oder mehrere Vertiefungen auf dem optischen und/oder nicht-optischen Teil bereitgestellt werden, in welchen die Datenspeichereinheit, insbesondere ein Datenspeicherelement und/oder eine Antennenstruktur, zumindest teilweise angeordnet werden. Gemäß einer optionalen Ausführungsform kann etwa eine Vertiefung in den optischen und/oder nicht-optischen Teil gefräst oder anderweitig eingebracht sein, in welche ein Datenspeicherelement, beispielsweise ein Chip, der Datenspeichereinheit eingebracht und befestigt wird. Dies bietet den Vorteil, dass ein Hervorstehen der Datenspeichereinheit von der Oberfläche des optischen und/oder nicht-optischen Teils reduziert oder vermieden werden kann.

Optional können die elektronisch auslesbaren Daten eine eindeutige Identifikationsbezeichnung zur Identifikation des ophthalmischen Implantats umfassen. Dies bietet den Vorteil, dass das ophthalmische Implantat auch dann identifizierbar ist, wenn dieses in einem Auge implantiert ist und möglicherweise zumindest teilweise verdeckt ist, beispielsweise durch die Iris, und/oder wenn das ophthalmische Implantat nicht für eine direkte physikalische elektrische Kontaktierung mittels eines elektrischen Leiters zugänglich ist.

Alternativ oder zusätzlich können die elektronisch auslesbaren Daten eine Information über eine oder mehrere Eigenschaften des Implantats, insbesondere eine optische Eigenschaft, eine Materialeigenschaft, eine Eigenschaft betreffend die Haptik-Plattform, eine Eigenschaft betreffend ein IOL-Design, und/oder eine Eigenschaft betreffend einen Herstellungsprozess, wie etwa ein Herstellungsdatum, des ophthalmischen Implantats aufweisen. Alternativ oder zusätzlich können die elektronisch auslesbaren Daten Informationen über die A-Konstante eines als IOL ausgebildeten ophthalmischen Implantats und/oder eine Information über eine torische Achse im Falle einer torischen IOL aufweisen. Dies bietet die Möglichkeit, durch ein Auslesen der jeweiligen Informationen die Eignung des Implantats für einen vorgesehenen Einsatz bzw. Patienten zweifelsfrei zu bestimmen.

Alternativ oder zusätzlich können die elektronisch auslesbaren Daten eine Information über den Patienten, in welchen das ophthalmische Implantat implantiert ist oder für welchen das ophthalmische Implantat vorgesehen ist aufweisen. Dadurch kann die Zuordnung des ophthalmischen Implantats vereinfacht und entsprechend etwaige fehlerhafte Zuordnungen vermieden werden.

Alternativ oder zusätzlich können die elektronisch auslesbaren Daten eine Information über die Implantation des ophthalmischen Implantats aufweisen, wie etwa ein Datum der Implantation und/oder die Benennung einer medizinischen Einrichtung und/oder von medizinischem Personal, welches in die Implantation involviert war.

Alternativ oder zusätzlich können die elektronisch auslesbaren Daten eine von einem mit dem ophthalmischen Implantat verbundenen Sensor bereitgestellte Information umfassen. Beispielsweise können ein Sensor zur Messung einer Zuckerkonzentration im Inneren des Auges und/oder ein Sensor zur Messung des Innendrucks des Auges mit dem ophthalmischen Implantat verbunden sein, sodass die von diesen Sensoren bereitgestellten Informationen über das ophthalmische Implantat auslesbar sind. Dadurch kann die Funktionalität des ophthalmischen Implantats hinsichtlich des kontaktlosen elektronischen Auslesens auch für andere Zwecke genutzt werden, die nicht notwendigerweise in direktem Zusammenhang mit dem ophthalmischen Implantat stehen.

Alternativ oder zusätzlich können die elektronisch auslesbaren Daten eine Netzwerkadresse und/oder Zugangsdaten zu einem über ein Netzwerk zugänglichen Datenspeicher aufweisen, an welcher Daten mit Informationen über das ophthalmische Implantat und/oder den Patienten hinterlegt und/oder hinterlegbar sind. Dies bietet den Vorteil, dass nicht alle Daten bzw. Informationen, welche dem ophthalmischen Implantat zugeordnet werden sollen, notwendigerweise auf der Datenspeichereinheit des ophthalmischen Implantats gespeichert werden müssen, sondern auch auf einem externen Server bereitgestellt werden können, welcher unter der von den Daten umfassten Netzwerkadresse erreichbar ist. Dies bietet den Vorteil, dass die Speicherkapazität der Datenspeichereinheit geringgehalten werden kann, da die Datenmenge, welche auf der Datenspeichereinheit gespeichert wird, auf ein Minimum reduziert werden kann. Auch bietet dies den Vorteil, dass die auf dem Server hinterlegten Daten auf einfache Weise gewartet und gegebenenfalls aktualisiert werden können, ohne dass ein Zugriff auf die Daten der Datenspeichereinheit erforderlich ist. Ein weiterer Vorteil besteht darin, dass Zugriffsrechte auf die auf dem Server hinterlegten Daten selektiv beschränkt werden können, sodass beispielsweise nur ein vorbestimmter Personenkreis von Berechtigten die Daten abrufen können.

Optional entspricht ein Elastizitätsmodul und/oder ein Wärmeausdehnungskoeffizient der kontaktlos elektronisch auslesbaren Datenspeichereinheit im Wesentlichen einem Elastizitätsmodul und/oder einem Wärmeausdehnungskoeffizienten des optischen Teils und/oder des nicht-optischen Teils. "Im Wesentlichen" bedeutet dabei, dass eine etwaige Abweichung zwischen dem Elastizitätsmodul und/oder dem Wärmeausdehnungskoeffizienten der Datenspeichereinheit und dem Elastizitätsmodul und/oder dem Wärmeausdehnungskoeffizienten des optischen und/oder nicht optischen Teils derart gering ist, dass sich durch die Datenspeichereinheit keine Einbußen für die Handhabbarkeit des ophthalmischen Implantats ergeben. Insbesondere sind optional die Elastizitätsmodule derart aufeinander abgestimmt, dass sich ein für die Implantation etwaig erforderliches Falten und Entfalten des ophthalmischen Implantats ohne Beeinträchtigung durch die Datenspeichereinheit umsetzen lässt. Alternativ oder zusätzlich können insbesondere die Wärmeausdehnungskoeffizienten derart aufeinander abgestimmt sein, dass bei auftretenden Temperaturänderungen am ophthalmischen Implantat, beispielsweise beim Sterilisieren, keine derart großen mechanischen Spannungen auftreten, welche zu einer irreversiblen Verformung oder Beeinträchtigung der Funktionalität des ophthalmischen Implantats führen würden.

Optional ist die kontaktlos elektronisch auslesbare Datenspeichereinheit mittels Verkleben und/oder Laminieren und/oder Verschweißen und/oder Umgießen auf den nicht-optischen und/oder den optischen Teil aufgebracht. Dies bietet den Vorteil, dass die Verbindung zwischen der Datenspeichereinheit und dem optischen Teil und/oder nicht-optischen Teil derart robust ausgestaltet werden kann, dass das Risiko einer Delamination und/oder einer anderen Form des Ablösens reduziert werden kann. Eine Anbringung der Datenspeichereinheit auf einer Oberfläche des optischen und/oder nicht-optischen Teils hat den Vorteil, dass das optische und/oder nicht-optische Teil einer möglichen thermischen Ausdehnung des Datenspeichers nicht im Wege steht. Dies ist vorteilhaft in der Sterilisationsphase und auch für die Lagerbedingungen. Zudem ist eine Faltung auf der Oberfläche geringer als im Kern des optischen und/oder nicht-optischen Teils. Das Aufbringen kann, wie bereits erwähnt, beispielsweise über ein Verkleben (z.B. mit flexiblem Acrylatkleber) erfolgen. Auch eine Einbettung der Datenspeichereinheit in eine Polymerhülle, welche optional flexibler ist als das Linsenmaterial (z.B. Silikon) ist via Lamination möglich. Das Aufbringen schließt dabei nicht aus, dass optional eine oder mehrere Vertiefungen in das ophthalmische Implantat eingebracht werden können, in welche die Datenspeichereinheit beim Aufbringen auf das ophthalmische Implantat zumindest teilweise eingeführt wird. Die Datenspeichereinheit kann somit optional auch in einem Bohrloch bzw. in einer Fräsung im optischen und/oder nicht-optischen Teil (z.B. bei dreiteiligen IOLs) versenkt und umgossen werden.

Optional weist die kontaktlos elektronisch auslesbare Datenspeichereinheit ein Datenspeicherelement und eine Antennenstruktur auf. Die Antennenstruktur und das Datenspeicherelement können separat ausgebildete Elemente darstellen, welche miteinander verbunden sind. Alternativ können diese einstückig ausgebildet sein, sodass die Antennenstruktur und das Datenspeicherelement zusammen einen Chip bilden.

Gemäß einer optionalen Ausführungsform ist die Antennenstruktur ausschließlich auf einer Haptik des nicht-optischen Teils angeordnet und weist optional eine Antennenfläche von 2 mm² bis 5 mm² auf. Dies bietet den Vorteil, dass keine Anordnung der Antennenstruktur auf dem optischen Teil erfolgt und somit eine Beeinträchtigung der optischen Eigenschaften bzw. der optischen Wirkung des optischen Teils durch die Antennenstruktur gänzlich vermieden werden kann. Ferner bietet dies den Vorteil, dass ein Risiko eines Bruchs und/oder anderweitiger Beschädigungen der Antennenstruktur reduziert werden kann, da sich die Antennenstruktur nicht im Bereich des Übergangs des nicht-optischen Teils zum optischen Teil des ophthalmischen Implantats erstreckt, an welchem erfahrungsgemäß häufig große mechanische Kräfte auftreten können. Vor allem können am Übergang des nicht-optischen Teils zum optischen Teil während des Faltens und/oder Implantierens des ophthalmischen Implantats große mechanische Kräfte auftreten, die zu einer unerwünschten Belastung der Antennenstruktur führen können, sofern diese in diesem Bereich verläuft. Optional können auch andere elektrische und/oder elektronische Komponenten auf dem nicht-optischen Teil des ophthalmischen Implantats angeordnet sein, auf welchem die Antennenstruktur angeordnet ist, wie etwa eine Datenspeichereinheit und/oder ein Datenspeicherelement. Optional sind alle elektrischen und/oder elektronischen Komponenten der Datenspeichereinheit auf dem nicht-optischen Teil des ophthalmischen Implantats angeordnet, auf welchem auch die Antennenstruktur angeordnet ist.

Gemäß einer weiteren optionalen Ausführungsform ist die Antennenstruktur ausschließlich auf einer oder mehreren Haptiken des nicht-optischen Teils und in einem peripheren Bereich des optischen Teils angeordnet und weist optional eine Antennenfläche von 6 mm² bis 30 mm² auf. Dies bietet den Vorteil, dass die Antennenfläche deutlich größer ausgebildet werden kann als bei einer Anordnung ausschließlich auf einer Haptik. Dadurch, dass im optischen Teil die Antennenstruktur lediglich in einem peripheren Bereich angeordnet ist, kann zudem eine Beeinträchtigung der optischen Wirkung des optischen Teils geringgehalten werden.

Gemäß einer weiteren optionalen Ausführungsform ist die Antennenstruktur ausschließlich derart an dem ophthalmischen Implantat angeordnet, dass die Antennenstruktur entlang einer äußeren Kontur des ophthalmischen Implantats verläuft und optional eine Antennenfläche von 6 mm² bis 30 mm² aufweist. Dies bietet den Vorteil, dass eine große Antennenfläche erzielt werden kann und zudem die Beeinträchtigung der optischen Wirkung des optischen Teils geringgehalten werden kann. Außerdem kann eine derartige Anordnung vorteilhaft hinsichtlich einer Stabilisierung des ophthalmischen Implantats wirken.

Gemäß einer weiteren optionalen Ausführungsform ist die Antennenstruktur ausschließlich derart angeordnet, dass die Antennenstruktur über die äußere Kontur des optischen Teils und nicht-optischen Teils des ophthalmischen Implantats, insbesondere über den optischen Teil und die Haptik(en) hinausragt und optional eine Antennenfläche von 30 mm² bis 100 mm² aufweist. Optional ist die Antennenstruktur ferner dazu ausgelegt, in einem in ein Auge implantierten Zustand des ophthalmischen Implantats den Kapselsack des Auges aufzuspannen. Dazu kann die Antennenstruktur optional selbsttragend ausgebildet sein, um eine geeignete Steifigkeit aufzuweisen. Alternativ oder zusätzlich kann die Antennenstruktur teilweise oder vollständig auf einem Kapselspannring angeordnet sein, der zum Aufspannen des Kapselsacks des Auges dient. Dabei kann beispielsweise der nicht-optische Teil den Kapselspannring aufweisen. Dies bietet demnach den Vorteil, dass eine große Antennenfläche erzielt werden kann, welche optional die Fläche des ophthalmischen Implantats übersteigen kann. Außerdem kann dabei die Antennenstruktur, beispielsweise nach Art einer mechanischen Feder, dazu eingesetzt werden, den Kapselsack zumindest teilweise aufzuspannen. Dadurch können sich demnach vorteilhafte Synergien durch mehrere Funktionalitäten der Antennenstruktur ergeben.

Optional ist die Antennenstruktur derart ausgebildet, dass diese den optischen Teil und/oder den nicht-optischen Teil, insbesondere eine Haptik des nicht-optischen Teils, mechanisch verstärkt und/oder stabilisiert. Die genannten optionalen Ausführungsformen mit den verschiedenen Anordnungen der Antennenstruktur bieten ferner den Vorteil, dass Antennenflächen von verschiedenen Größen realisiert werden können. Dadurch kann beispielsweise die Sensitivität der Antennenstruktur an vorgegebene Eigenschaften angepasst werden, beispielsweise an die zu erwartende Sendeleistung eines Lesegerätes und/oder an den zu erwartenden Abstand des Lesegerätes von der Antennenstruktur. Prinzipiell kann mit einer größeren Antennenfläche eine größere Sensitivität erreicht werden.

Optional weist die Antennenstruktur eine oder mehrere Leiterbahnen auf, welche zumindest teilweise in geradlinig verlaufenden parallelen Windungen verlaufen und/oder zumindest teilweise mäanderförmig und/oder wellenförmig verlaufen. Dies bietet eine Flexibilität hinsichtlich der mechanischen Eigenschaften der Leiterbahnen. Insbesondere können mäanderförmige Windungen den Vorteil bieten, dass die Leiterbahnen durch mechanischen Zug und/oder Druck dehnbar und/oder stauchbar sind. Dies kann insbesondere für solche ophthalmischen Implantate vorteilhaft sein, welche beispielsweise während der Implantation stark verformt werden, etwa durch ein Entfalten nach der Einfügung in einen Kapselsack, und/oder während der bestimmungsgemäßen Verwendung in implantiertem Zustand verformt werden, wie etwa bei für die Akkommodation verformbaren IOLs. Optional weist das ophthalmische Implantat nur solche Materialien auf, welche eine gleiche oder ähnliche Elastizität aufweisen und/oder ein gleiches oder ähnliches Reaktionsverhalten bzw. Dehnungsverhalten bei einwirkende Kräfte, Drücke und Wärme. Dabei können etwa die Elastizitätsmodule der Materialien im Druckbereich von 0,5 MPa bis 3 MPa bei einer Poissonzahl im Bereich 0,3 bis 0,49 liegen. Die Wärmeausdehnungskoeffizienten können beispielsweise im Bereich von 0,05 mm/m/K bis 0,1 mm/m/K liegen.

Optional weist der nicht-optische Teil zumindest eine armförmige Haptik auf und ist die Antennenstruktur zumindest teilweise in einem Bereich der zumindest einen armförmigen Haptik angeordnet, der bei einer gebrauchsüblichen Verformung der armförmigen Haptik die geringsten mechanischen Spannungen aufweist. Beispielsweise kann ein innerer Teil des Arms der Haptik bei einer Verformung des Arms geringeren mechanischen Spannungen ausgesetzt sein, als die äußeren Teile des Arms. Entsprechend kann eine Anordnung der Antennenstruktur im inneren Teil des Arms der Haptik den Vorteil bieten, dass die mechanischen Belastungen der Antennenstruktur geringer gehalten werden können, als dies bei einer anderen Anordnung der Antennenstruktur auf der Haptik möglich wäre.

Optional ist die Antennenstruktur derart ausgebildet, dass diese in einem in ein Auge implantierten Zustand des ophthalmischen Implantats zumindest teilweise den Kapselsack des Auges aufspannt oder ein Aufspannen des Kapselsacks durch das ophthalmische Implantat unterstützt. Beispielsweise kann der nicht-optische Teil eine dreidimensionale Struktur bzw. Haptik aufweisen, welche zum Aufspannen des Kapselsacks vorgesehen ist. Die Antennenstruktur ist optional derart ausgebildet ist, dass in implantiertem Zustand des ophthalmischen Implantats die Antennenstruktur eine periodische Strukturierung der Antennenoberfläche und/oder Kanten aufweist. Die periodische Strukturierung und/oder die Kanten können dabei eine mechanische Reibung zwischen der Antennenstruktur auf der Oberfläche des ophthalmischen Implantats und dem Kapselsack und/oder einem anderen Teil des Augengewebes verursachen, welche eine Relativbewegung des ophthalmischen Implantats und des Auges, insbesondere ein gegenseitiges Verrutschen, erschweren und demnach die Positionierung des ophthalmischen Implantats im Auge stabilisieren.

Optional ist die Antennenstruktur aus einem solchen Material gefertigt, dass dieses ein Anhaften von Zellen erschwert. Insbesondere kann die Antennenstruktur eine Oberflächenenergie aufweisen, welche von der Oberflächenenergie des optischen und/oder nicht-optischen Teils abweicht und dadurch dem Anhaften und/oder Wachstum von Zellen entgegenwirkt. Dadurch kann einer Entstehung eines Nachstars zumindest teilweise vorgebeugt werden. Ein geeignetes Verhältnis der Oberflächenenergie des optischen und/oder nicht-optischen Teils zur Oberflächenenergie der Antennenstruktur kann durch eine geeignete Wahl der Materialen für den optischen und/oder nicht-optischen Teil und der Antennenstruktur erzielt werden.

Optional umfasst die kontaktlos elektronisch auslesbare Datenspeichereinheit ein RFID-Tag oder ist als solches ausgebildet. Dies bietet den Vorteil, dass eine Energieversorgung der Datenspeichereinheit bei Bedarf durch ein externes Lesegerät bereitgestellt werden kann und entsprechend eine integrierte Energieversorgung nicht zwingend erforderlich ist.

Optional ist die kontaktlos elektronisch auslesbare Datenspeichereinheit mittels eines separaten Lesegerätes derart kontaktlos auslesbar, dass ein Abstand der kontaktlos elektronisch auslesbaren Datenspeichereinheit zum Lesegerät während eines Auslesevorgangs optional im Bereich 5 mm bis 300 mm, optional 10 mm bis 200 mm, optional 25 mm bis 75 mm wählbar ist. Dies bietet den Vorteil, dass für den Auslesevorgang das Lesegerät nicht in mechanischen Kontakt mit dem Auge gebracht werden muss, wenngleich dies gemäß manchen Ausführungsformen möglich sein kann.

Optional ist die kontaktlos elektronisch auslesbare Datenspeichereinheit dazu ausgelegt, elektronische Signale zu empfangen und/oder zu senden, welche in einem bestimmten Frequenzbereich zwischen 100 kHz und 6 GHz liegen. Insbesondere kann für die Kommunikation mit einem Lesegerät solch ein Frequenzbereich gewählt werden, der für die Verwendung in medizinischen Produkten zugelassen und/oder geeignet und/oder empfohlen ist.

Tabelle 1 nennt beispielhaft verschiedene Frequenzen und Frequenzbänder, welche typischerweise bei verschiedenen Arten von intrakorporalen Medizingeräten zur Anwendung kommen und welche optional auch für ein ophthalmisches Implantat gemäß der vorliegenden Offenbarung verwendet werden können.

**Tabelle1:**

| **Intrakorporale Medizingeräte** | **Frequenz(en)** |
|---|---|
| implantierbare Medizingeräte | 402 MHz |
| | 433 MHz |
| | 868 MHz |
| | 915 MHz |
| | 1,4 GHz |
| | 2,45 GHz |
| | Ultrabreitband (UWB) um 6 GHz |
| einnehmbare Medizingeräte | 433 MHz |
| | 500 MHz |
| | 800 MHz |
| | 1,2 GHz |
| | 1,4 GHz |
| | 2,4 GHz |
| injizierbare Medizingeräte | 132 KHz |
| | 2 MHz |
| | 13,56 MHz |
| | 915 MHz |

Optional kann demnach die Datenspeichereinheit dazu ausgelegt sein, für das kontaktlose elektronische Auslesen der Daten eine Frequenz von 13,56 MHz zu verwenden. Diese Frequenz kann einen vorteilhaften Kompromiss zwischen Antennengröße, Arbeitsabstand zum Lesegerät und zu erwartenden Verlusten bzw. Abschwächungen des Signals in Luft und Gewebe für ein ophthalmisches Implantat darstellen.

Tabelle 2 nennt beispielhaft verschiedene Antennendesigns, welche typischerweise bei verschiedenen Arten von intrakorporalen Medizingeräten zur Anwendung kommen und welche optional auch für ein ophthalmisches Implantat gemäß der vorliegenden Offenbarung verwendet werden können.

**Tabelle 2**

| **Intrakorporale Medizingeräte** | **Antennendesigns** |
|---|---|
| implantierbare Medizingeräte | PIFA |
| | Patch |
| | Loop |
| | Monopole |
| einnehmbare Medizingeräte | Helica |
| | Spiral |
| | PIFA |
| injizierbare Medizingeräte | Loop |
| | Dipole |

Optional können der optische Teil und/oder der nicht-optische Teil aus mehreren Elementen, d.h. mehrteilig, ausgebildet sein. Die jeweils mehreren Elemente können beim Implantationsprozess zusammengesetzt, d.h. miteinander verbunden werden, sodass die mehreren Elemente in verbundenem Zustand den optischen Teil bzw. den nicht-optischen Teil bilden und insbesondere das ophthalmische Implantat bilden. Optional kann das ophthalmische Implantat mehrere verbindbare Elemente umfassen, welche separat voneinander vorliegen und beim Implantationsprozess miteinander verbunden werden. Beispielsweise kann ein Element des Implantats zumindest ein Teilstück des nicht-optischen Teils und zumindest ein Teilstück des optischen Teils umfassen, sodass die Teile in verbundenem Zustand das Implantat bilden. Gemäß einer optionalen Ausführungsform weist eines der Elemente den gesamten nicht-optischen Teil sowie ein Teilstück des optischen Teils auf und das andere Element das verbleibende Teilstück des optischen Teils. Während also in nicht-verbundenem Zustand ein Element unter anderem den gesamten nicht-optischen Teil bildet, wird der optische Teil erst mit verbinden der beiden Elemente komplettiert. Dies kann den Implantationsprozess erleichtern. In ähnlicher Weise kann gemäß einer anderen optionalen Ausführungsform ein Element den gesamten optischen Teil und ein Teilstück des nicht-optischen Teils bilden, während das andere Element das verbleibende Teilstück des nicht-optischen Teils bildet.

Optional ist das ophthalmische Implantat derart ausgebildet, dass die Datenspeichereinheit um den optischen Teil des ophthalmischen Implantats herum angeordnet ist. Dabei kann die Datenspeichereinheit eine als Ringantenne ausgebildete Antennenstruktur aufweisen, welche um den optischen Teil des ophthalmischen Implantats herum angeordnet ist und optional den optischen Teil des ophthalmischen Implantats umschließt. Optional kann auch das Datenspeicherelement nahe des Randes des optischen Teils ausgebildet sein. Dies bietet den Vorteil, dass die gesamte Datenspeichereinheit in kompakter Weise nahe am optischen Teil angeordnet werden kann. "Nahe des Randes des optischen Teils" bedeutet dabei, dass ein Abstand zum Rand des optischen Teils nicht größer als etwa 1 mm ist.

Die oben genannten und im Folgenden erläuterten Merkmale und Ausführungsformen sind dabei nicht nur als in den jeweils explizit genannten Kombinationen offenbart anzusehen, sondern sind auch in anderen technisch sinnhaften Kombinationen und Ausführungsformen vom Offenbarungsgehalt umfasst.

Weitere Einzelheiten und Vorteile sollen nun anhand von den folgenden Beispielen und optionalen Ausführungsformen mit Bezug auf die Figuren näher erläutert werden.

Es zeigen:
- Figur 1: herkömmliche Kennzeichnungen von als Intraokularlinsen ausgeführten ophthalmischen Implantaten;
- Figur 2: in einer schematischen Darstellung verschiedene Szenarien, bei welchen eine eindeutige Identifikation des ophthalmischen Implantats wünschenswert sein kann;
- Figuren 3A bis 3H: verschiedene optionale Ausführungsformen von ophthalmischen Implantaten, die als Intraokularlinsen ausgebildet sind und jeweils verschiedenartig ausgebildete Datenspeichereinheiten mit einer Antennenstruktur aufweisen;
- Figuren 4A und 4B: verschiedene beispielhafte Anordnungen von Leiterbahnen einer Antennenstruktur eines ophthalmischen Implantats;
- Figur 5: eine Modellierung einer Haptik eines nicht-optischen Teils einer Intraokularlinse, welche die auftretende mechanische Spannung in der Haptik bei einem bestimmungsgemäßen Gebrauch der Intraokularlinse in implantiertem Zustand erkennen lässt.

In den folgenden Figuren werden gleiche oder ähnliche Elemente in den verschiedenen Ausführungsformen der Einfachheit halber mit gleichen Bezugszeichen bezeichnet.

Figur 1 zeigt beispielhaft herkömmliche Kennzeichnungen von als Intraokularlinsen ausgeführten ophthalmischen Implantaten. Diese stellen eine Verpackungskennzeichnung 10 für Intraokularlinsen und einen Patienten Implantationspass 18 dar. Insbesondere zeigt die Verpackungskennzeichnung 10 das Herstellungsdatum 12 in einem Datumsformat gemäß ISO 8601, einen Geräteidentifikationscode 14 (Device Identifier, DI, GTIN, product identifier), sowie einen Produktionsidentifikationscode 16 (Production Identifier, PI, Application Identifiers), welcher beispielsweise eine Seriennummer, eine Losnummer und/oder ein Ablaufdatum beinhaltet. Die gezeigten Kennzeichnungen lassen erkennen, welche Informationen typischerweise zur Kennzeichnung von ophthalmischen Implantaten bereitgestellt werden. Außerdem lässt die Verpackungskennzeichnung 10 erkennen, dass diese Informationen herkömmlicherweise nicht direkt an dem ophthalmischen Implantat angebracht sind, sondern vielmehr an einer Verpackung des ophthalmischen Implantats oder auf einem separaten Patientenpass. Diese Formen der Anbringung weisen den Nachteil auf, dass eine eindeutige Zuordnung und Identifikation des ophthalmischen Implantats verloren gehen bzw. unmöglich werden kann, insbesondere wenn das ophthalmische Implantat aus der Verpackung entnommen ist und etwa in ein Auge implantiert wurde.

Figur 2 zeigt in einer schematischen Darstellung verschiedene Szenarien, bei welchen eine eindeutige Identifikation des ophthalmischen Implantats wünschenswert sein kann. Gemäß dem erläuterten Szenario sind Informationen zum ophthalmischen Implantat auf einem Server 22 bzw. in einer Cloud abgelegt, welcher über ein Netzwerk erreichbar ist. Eine Identifikation des ophthalmischen Implantats kann beispielsweise während der Operation zur Implantation des ophthalmischen Implantats erfolgen, etwa durch ein Operationsmikroskop 24. Dabei kann das Operationsmikroskop ein Lesegerät aufweisen, welches dazu geeignet ist, Daten kontaktlos elektronisch von einer Datenspeichereinheit des ophthalmischen Implantats auszulesen. Dies kann beispielsweise dazu dienen, eine richtige Zuordnung des ophthalmischen Implantats zum Patienten zu überprüfen und/oder Eigenschaften über das ophthalmische Implantat, welche etwa für die Implantation hilfreich oder erforderlich sind, in Erfahrung zu bringen. Dazu kann beispielsweise eine Information zur Identifikation des ophthalmischen Implantats und/oder eine Netzwerkadresse zum Server von der Datenspeichereinheit ausgelesen werden und die weiteren Informationen vom Server bezogen werden.

Auch nach der Operation, wenn das Implantat bereits implantiert ist, können sich verschiedene Szenarien ergeben, in denen eine Identifikation und/oder ein Abrufen von Informationen über das ophthalmische Implantat vorteilhaft ist. Dies kann insbesondere bei Augenuntersuchungen der Fall sein. Entsprechend können beispielsweise medizinische Geräte, welche für die Untersuchung der Augen verwendet werden, ebenfalls mit einem entsprechenden Lesegerät versehen sein, um die Daten elektronisch und kontaktlos aus der Datenspeichereinheit des ophthalmischen Implantats auszulesen. Figur 2 zeigt dazu beispielhaft ein Operationsmikroskop 24, welches während des Implantationsvorgangs zum Einsatz kommen kann, sowie ein Biometriegerät 26, eine Spaltlampe 28 und ein Autorefraktometer 30, welche in post-operativen Anwendungen eingesetzt werden können.

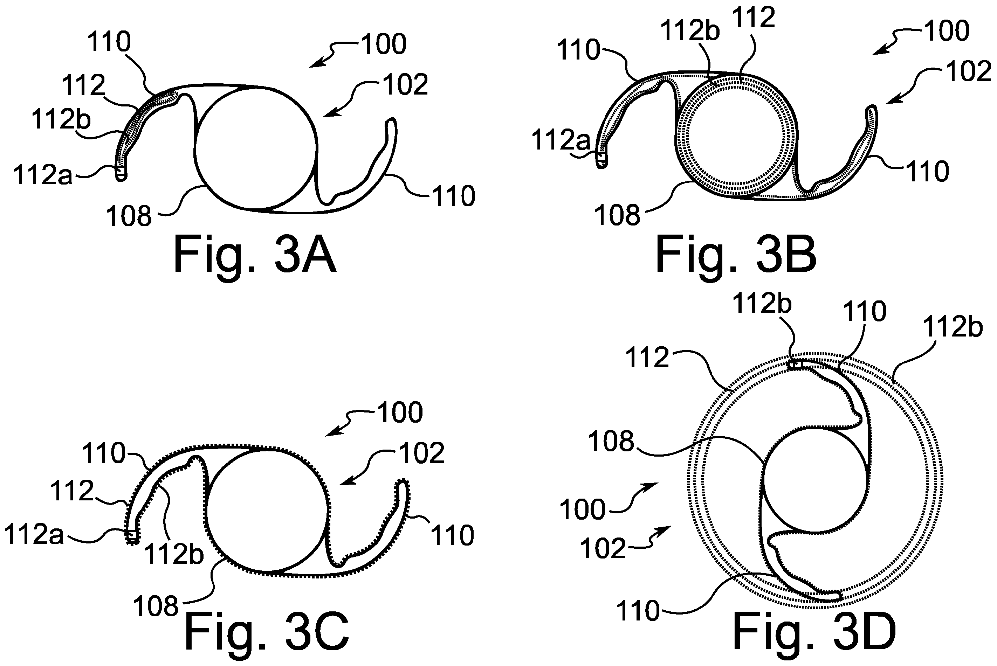

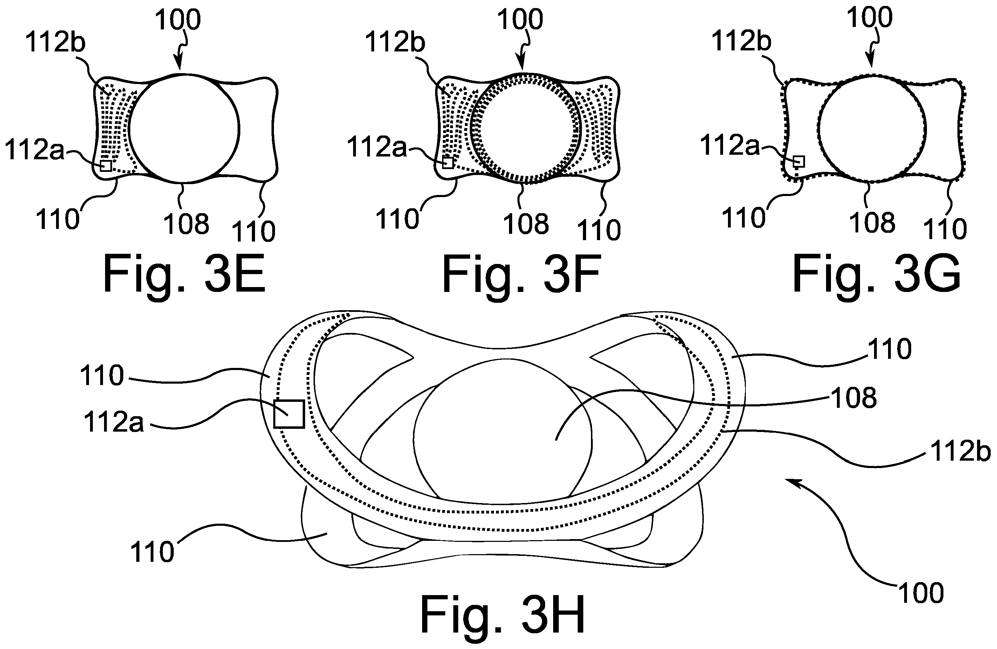

Die Figuren 3A bis 3D zeigen verschiedene optionale Ausführungsformen von ophthalmischen Implantaten 100, die als Intraokularlinsen 102 ausgebildet sind und jeweils verschiedenartig ausgebildete Datenspeichereinheiten 112 mit einer Antennenstruktur 112b aufweisen.

Die ophthalmischen Implantate 100 bzw. Intraokularlinsen 102 weisen jeweils einen optischen Teil 108 und einen nicht-optischen Teil auf 110. Der optische Teil 108 wird dabei durch eine optische Linse gebildet, welche eine refraktive und/oder diffraktive Wirkung aufweist. Der nicht-optische Teil 110 umfasst zwei Haptiken, welche an dem optischen Teil 108 verbunden sind, eine armförmige Struktur aufweisen und sich vom optischen Teil wegerstrecken. Der nicht-optische Teil weist dabei keine optische Wirkung auf und muss nicht zwingend optisch transparent ausgebildet sein.

Die ophthalmischen Implantate 108 weisen ferner eine Datenspeichereinheit 112 auf, welche wiederum ein Datenspeicherelement 112a und eine Antennenstruktur 112b aufweist, die mit dem Datenspeicherelement 112a verbunden ist. Die Datenspeichereinheit 112 ist dabei auf den optischen und/oder nicht-optischen Teil des ophthalmischen Implantats aufgebracht. Beispielsweise kann die Datenspeichereinheit 112 nach Fertigstellung des optischen und nicht-optischen Teils aufgebracht worden sein. Die Datenspeichereinheit kann auf dem Datenspeicherelement 112a Daten speichern bzw. aufweisen, welche kontaktlos elektronisch mittels eines geeigneten Lesegerätes auslesbar sind. Dies kann beispielsweise mittels elektromagnetischer Wellen erfolgen, welche über die Antennenstruktur 112b von der Datenspeichereinheit 112 empfangen und gesendet werden können. Auch kann die Datenspeichereinheit 112 dazu ausgelegt sein, über die Antennenstruktur 112b mit Energie versorgt zu werden, welche beispielsweise über Induktion kontaktlos vom Lesegerät bereitgestellt wird. Die Datenspeichereinheit kann insbesondere als ein RFID Tag bzw. RFID Chip ausgebildet sein oder einen solchen umfassen. Derartige ophthalmische Implantate 100 bieten den Vorteil gegenüber solchen mit optischen Markierungen, dass ein kontaktloses elektronisches Auslesen auch dann möglich ist, wenn die Datenspeichereinheit 112 in implantiertem Zustand teilweise oder vollständig verdeckt ist, beispielsweise von der Iris des Auges.

Die verschiedenen Ausführungsformen, welche in den Figuren 3A bis 3D dargestellt sind, unterscheiden sich dabei durch unterschiedlich ausgebildete Datenspeichereinheiten 112. Diese weichen insbesondere in der Ausgestaltung und/oder Anordnung der Antennenstruktur 112b voneinander ab, welche im Folgenden erläutert werden.

Gemäß der Ausführungsform in Figur 3A ist die Datenspeichereinheit 112 vollständig auf einer der Haptiken des nicht-optischen Teils 110 angeordnet. Insbesondere ist die Datenspeichereinheit auf der führenden Haptik der Intraokularlinsen 102 angeordnet. Wie in Figur 3A erkennbar ist, sind das Datenspeicherelement 112a und auch die gesamte Antennenstruktur 112b auf der selben Haptik angeordnet. Die Antennenstruktur verläuft in einer schleifenförmigen Anordnung mit mehreren Schleifen ausschließlich auf der einen Haptik. Auch eine Anordnung in lediglich einer Schleife ist möglich. Dabei kann die Antennenstruktur 112b eine Antennenfläche von etwa 3,5 mm² bis 4 mm² aufweisen. Diese Ausführungsform bieten den Vorteil, dass die Datenspeichereinheit ausschließlich auf dem nicht-optischen Teil 110 und insbesondere auf einer einzigen Haptik angeordnet sein kann und entsprechend keine Anordnung der Datenspeichereinheit 112 auf dem optischen Teil erforderlich ist.

Gemäß der in Figur 3B gezeigten Ausführungsform erstreckt sich die Datenspeichereinheit 112 sowohl über beide Haptiken des nicht-optischen Teils als auch über einen peripheren Bereich des optischen Teils. Dabei ist das Datenspeicherelement 112a, wie auch bei der vorhergehenden Ausführungsform, auf einer Haptik angeordnet. Allerdings erstreckt sich die Antennenstruktur über beide Haptiken und auch über den peripheren Bereich des optischen Teils 108, wobei mehrere Schleifen um den peripheren Bereich des optischen Teils 108 herumlaufen. Dies bietet den Vorteil, dass eine große Antennenfläche erzielt werden kann, beispielsweise etwa 30 mm².

Gemäß der in Figur 3C gezeigten Ausführungsform ist die Antennenstruktur 112b derart angeordnet, dass diese einmal die Kontur der Intraokularlinse 102 umläuft. Dabei umläuft die Antennenstruktur 112b die Intraokularlinse nur einmal, wenngleich auch mehrere Umläufe möglich sind. Eine derartige Anordnung bietet den Vorteil, dass eine große Antennenfläche, etwa 10 mm² erzielt werden kann und dennoch eine Überlagerung des optischen Teils 108 durch die Antennenstruktur 112b auf ein Minimum und auf den äußersten peripheren Bereich begrenzt werden kann. Außerdem bietet die Anordnung den Vorteil, dass die Antennenstruktur zur mechanischen Stabilisierung der Intraokularlinse 102 beitragen kann.

Gemäß der in Figur 3D gezeigten Ausführungsform ist die Datenspeichereinheit 112 derart auf das ophthalmische Implantat aufgebracht, dass diese teilweise über das ophthalmische Implantat hinausragt. Insbesondere ist das Datenspeicherelement 112a auf eine der Haptiken der Intraokularlinse 102 aufgebracht und die Antennenstruktur 112b an jeder der beiden Haptiken befestigt. Allerdings erstreckt sich die Antennenstruktur 112b deutlich über den optischen und nicht-optischen Teil hinaus und spannt gemäß der gezeigten Ausführungsform einen Kreisbogen auf. Dies bietet den Vorteil, dass eine deutlich größere Antennenfläche erzielt werden kann, etwa bis zu 100 mm², und entsprechend eine hohe Sensitivität der Datenspeichereinheit 112 erzielt werden kann. Außerdem bietet eine derartige Ausgestaltung der Antennenstruktur 112b die Möglichkeit, im implantierten Zustand mittels der Antennenstruktur 112b den Kapselsack des Auges aufzuspannen bzw. mechanisch zu stabilisieren.

Die Figuren 3E bis 3G zeigen weitere optionale Ausführungsformen von ophthalmischen Implantaten 100. Auch diese sind als IOL ausgebildet und weichen von den Ausführungsformen der Figuren 3A bis 3D in der Form ihrer Haptiken ab. Mit anderen Worten weisen diese ophthalmischen Implantate 100 jeweils eine nicht-optischen Teil 110 auf, der anders geformt ist, als der nicht-optische Teil 100 der Ausführungsformen in den Figuren 3A bis 3D. Auch bei dieser Formgebung ist in manchen Ausführungsformen die Datenspeichereinheit 112 nur im nicht-optischen Teil 110 angeordnet und in anderen Ausführungsformen sowohl im optischen Teil 108 als auch im nicht-optischen Teil 110.

Figur 3H zeigt ein ophthalmisches Implantat gemäß einer weiteren optionalen Ausführungsform, welches als IOL ausgebildet ist. Dabei weist der nicht-optische Teil dreidimensional geformte Haptiken auf, welche entlang der Richtung der optischen Achse voneinander beabstandete Haptiken bzw. Elemente aufweist. Dies bietet den Vorteil, dass mittels der Hapitk(en) beispielsweise in implantiertem Zustand der Kapselsack zumindest teilweise dreidimensional aufgespannt werden kann. Dabei können die Haptiken optional mechanisch durch die Antennenstruktur 112b verstärkt werden. Dadurch kann ein größeres Volumen im Kapselsack bereitgestellt werden und entsprechend ein Durchfluss des Kammerwassers ermöglicht bzw. erhöht werden. Mit anderen Worten kann dadurch ein "offener" Kapselsack bereitgestellt werden.

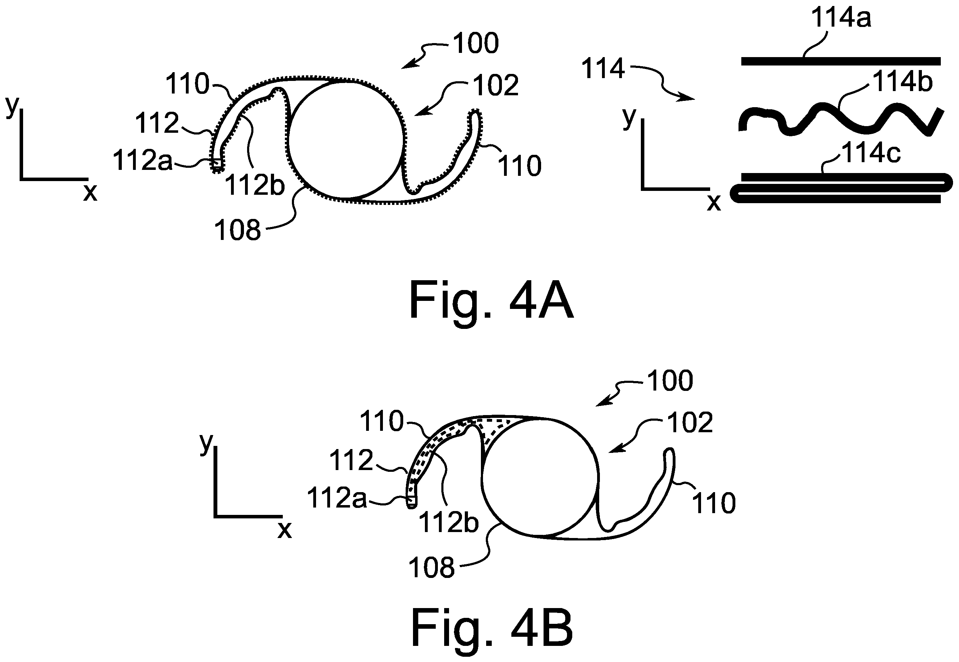

Figur 4A zeigt beispielhaft verschiedene Anordnungen von Leiterbahnen 114 einer Antennenstruktur 112b eines ophthalmischen Implantats 100. Dabei sind die Dimensionen in der Ebene des ophthalmischen Implantats durch die beispielhaft dargestellten Koordinatensysteme mit x und y bezeichnet. Gemäß den Darstellungen in Figur 4A bestehen verschiedene Möglichkeiten für die Anordnungen der Leiterbahnen 114 der Antennenstruktur 112b. Gemäß einer ersten Möglichkeit wird die Antennenstruktur 112b durch eine einzelne, geradlinig verlaufende Leiterbahn 114a gebildet, was zu einer besonders einfachen Herstellbarkeit der Antennenstruktur und einem besonders geringen Platzbedarf auf dem optischen Teil 108 und/oder nicht-optischen Teil 110 des ophthalmischen Implantats 100 führt. Dies bietet ferner den Vorteil, dass ein Verbindungsaufbau zwischen einem Externen Schreib-Lese-Gerät und der Datenspeichereinheit von vorne erleichtert wird, da die Spulenfläche im Wesentlichen senkrecht zur optischen Achse der ophthalmischen Linse und entsprechend im implantierten Zustand senkrecht zur optischen Achse des Auges angeordnet ist. "Von vorne" bedeutet dabei, dass sich das Schreib-Lese-Gerät an einer Position vor der Kornea des Auges befindet. Gemäß einer weiteren Ausführungsform wird die Antennenstruktur 112b durch eine einzelne Leiterbahn 114b gebildet, welche mäanderförmig bzw. in einer Wellenform verläuft. Dies bietet den Vorteil, dass die effektive Antennenfläche vergrößert werden kann und zudem eine mechanische Flexibilität hinsichtlich einer möglichen Stauchung und/oder Streckung erzielt werden kann. Letzteres kann insbesondere hinsichtlich einer möglicherweise erforderlichen Faltung und Entfaltung des ophthalmischen Implantats 100 bei der Implantation vorteilhaft sein. In einer weiteren Ausführungsform wird die Antennenstruktur 112b durch mehrere parallel in Schleifen verlaufende Leiterbahnen 114c gebildet. Dies bietet den Vorteil, dass eine effektive Antennenfläche durch mehrere Schleifen vergrößert werden kann. Eine größere Länge der Antennenstruktur 112b und/oder eine größere Anzahl von Schleifen kann dabei optional die Leistungsfähigkeit der Antennenstruktur 112b erhöhen.

Gemäß weiteren Ausführungsformen können Antennenstrukturen auch mit mehreren Anordnungen der Leiterbahnen bereitgestellt werden, sodass die Antennenstruktur 112b mehrere Abschnitte mit unterschiedlichen Anordnungen der Leiterbahn(en) aufweist.

Figur 4B zeigt eine weitere optionale Ausführungsform, welche sich dadurch von der in Figur 4A gezeigten Ausführungsform unterscheidet, dass sich die Antennenstruktur 112b nur im Bereich einer Haptik des nicht-optischen Bereichs 110 erstreckt, und anders als in der in Figur 4A gezeigten Ausführungsform nicht den optischen Teil 108 umläuft und nicht in dem Bereich der zweiten Haptik verläuft. Dies bietet den Vorteil, dass die Antennenstruktur 112 nicht im Bereich des Übergangs des nicht-optischen Teils 110 zum optischen Teils 108 ausgebildet ist, in welchem insbesondere beim Falten und Entfalten des ophthalmischen Implantats große mechanische Kräfte und entsprechend hohe Belastungen für die Antennenstruktur 112 auftreten können.

Im Allgemeinen ist die Verlaufsart der Leiterbahnen 114c der Antennenstruktur 112b unabhängig von der genauen Anordnung der Antennenstruktur 112b in und/oder auf dem ophthalmischen Implantat. Dies bedeutet, dass ein geradliniger, mäanderförmiger und/oder schleifenartiger Verlauf der Leiterbahnen unabhängig davon gewählt werden kann, ob sich die Antennenstruktur nur innerhalb eines nicht-optischen Bereichs 110 oder im und/oder um den optischen Bereichs 108 des ophthalmischen Implantats 100 erstreckt. Auch sind Antennenstrukturen 112b möglich, bei denen die Leiterbahnen 114c in unterschiedlichen Abschnitten unterschiedliche Verlaufsarten aufweisen. Die in Figur 4A gezeigten Verlaufsarten der Leiterbahnen 114c sind daher nicht zwingend an die in Figur 4A und Figur 4B beispielhaft gezeigten Anordnungen der Antennenstruktur 112b auf dem ophthalmischen Implantat gekoppelt. Ebenso kann die Verlaufsart der Leiterbahnen 114c derart ausgebildet sein, dass diese auch in z-Richtung, also parallel zur optischen Achse des Auges (in implantiertem Zustand) und/oder zur optischen Achse des ophthalmischen Implantats eine Variation aufweist. Beispielsweise können auch in der z-Richtung ein mäanderförmiger Verlauf und/oder ein wellenförmiger Verlauf und/oder einen schleifenförmigen Verlauf der Antennenstruktur 112b ausgebildet sein. Dies kann vorteilhaft sein, um einen Verbindungsaufbau zwischen einem Externen Schreib-Lese-Gerät und der Datenspeichereinheit von der Seite, d.h. senkrecht zur optischen Achse des Auges (in implantiertem Zustand) und/oder senkrecht zur optischen Achse des ophthalmischen Implantats zu erleichtern.

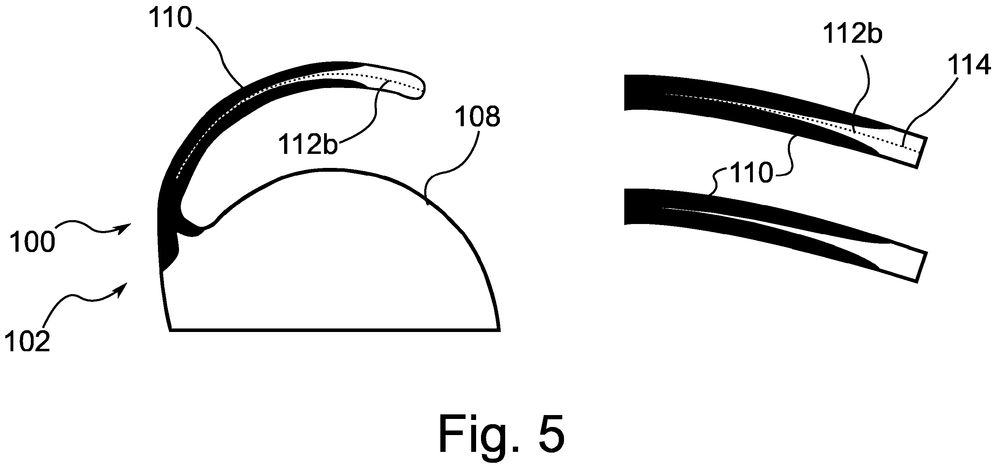

Figur 5 zeigt eine Modellierung einer Haptik eines nicht-optischen Teils 110 einer Intraokularlinse, welche die auftretende mechanische Spannung in der Haptik bei einem bestimmungsgemäßen Gebrauch der Intraokularlinse in implantiertem Zustand erkennen lässt. Dabei ist erkennbar, dass insbesondere in den Randbereichen der armförmigen Haptik bei Druckbelastung der Haptik große mechanische Spannungen auftreten, während ein innenliegender, mittlerer Bereich weitgehend frei von Spannungen ist. Entsprechend schlägt diese Ausführungsform das Aufbringen der Antennenstruktur 112b in dem, mittleren Bereich der Haptik vor, um eine unerwünschte Verformung der Antennenstruktur bei mechanischer Belastung der Haptik zu minimieren. Auf der rechten Seite ist eine Anordnung der Leiterbahn bzw. der Antennenstruktur 112b in der Mitte der Haptik im Detail gezeigt. Beispielsweise kann die Antennenstruktur 112b durch eine Leiterbahn 114 aus einem Kupferdraht mit einer Querschnittsfläche von 20 µm x 20 µm gebildet werden, der an der Haptik befestigt ist. Die Verformung der Leiterbahn bei mechanischer Verformung der Haptik ist bei einer derartigen Anordnung sehr gering und insbesondere deutlich geringer als die plastische Verformbarkeit des Kupferdrahtes, der die Leiterbahn 114 bildet.

### Bezugszeichenliste

- 10: Verpackungskennzeichnung
- 12: Herstellungsdatum
- 14: Geräteidentifikationscode
- 16: Produktionsidentifikationscode
- 18: Implantationspass
- 22: Server
- 24: Operationsmikroskop
- 26: Biometriegerät
- 28: Spaltlampe
- 30: Autorefraktometer

- 100: ophthalmisches Implantat
- 102: Intraokularlinse
- 108: optischer Teil
- 110: nicht-optischer Teil
- 112: Datenspeichereinheit
- 112a: Datenspeicherelement
- 112b: Antennenstruktur
- 114: Leiterbahn

## Patentansprüche

1. Ophthalmisches Implantat (100), umfassend:
- einen optischen Teil (108) mit einer optischen Wirkung;
- einen mit dem optischen Teil (108) verbundenen oder verbindbaren nicht-optischen Teil (110);
- eine im implantierten Zustand des ophthalmischen Implantats (100) kontaktlos elektronisch auslesbare Datenspeichereinheit (112), welche kontaktlos elektronisch auslesbare Daten enthält, anhand welcher das ophthalmische Implantat (100) identifizierbar ist;
wobei die elektronisch auslesbare Datenspeichereinheit (112) auf den nicht-optischen (110) und/oder den optischen Teil (108) aufgebracht ist und derart angeordnet ist, dass die optische Wirkung des optischen Teils (108) im Wesentlichen nicht durch die elektronisch auslesbare Datenspeichereinheit (112) beeinträchtigt wird.
**dadurch gekennzeichnet, dass**
ein Elastizitätsmodul und/oder ein Wärmeausdehnungskoeffizient der kontaktlos elektronisch auslesbaren Datenspeichereinheit (112) im Wesentlichen einem Elastizitätsmodul und/oder einem Wärmeausdehnungskoeffizienten des optischen Teils (108) und/oder des nicht-optischen Teils (110) entspricht.

2. Ophthalmisches Implantat (100) gemäß Anspruch 1, wobei die kontaktlos elektronisch auslesbare Datenspeichereinheit (112) ein Datenspeicherelement und eine Antennenstruktur aufweist.

3. Ophthalmisches Implantat (100) gemäß Anspruch 2, wobei die Antennenstruktur (112b)
- ausschließlich auf einer Haptik des nicht-optischen Teils (110) angeordnet ist und optional eine Antennenfläche von 2 mm² bis 5 mm² aufweist; oder
- auf einer oder mehreren Haptiken des nicht-optischen Teils (110) und in einem peripheren Bereich des optischen Teils (110) angeordnet ist und optional eine Antennenfläche von 6 mm² bis 30 mm² aufweist; oder
- derart an dem ophthalmischen Implantat (100) angeordnet ist, dass die Antennenstruktur (112b) entlang einer äußeren Kontur des ophthalmischen Implantats (100) verläuft und optional eine Antennenfläche von 6 mm² bis 30 mm² aufweist; oder
- derart angeordnet ist, dass die Antennenstruktur (112b) über die äußere Kontur des optischen Teils (108) und des nicht-optischen Teils (110) des ophthalmischen Implantats (100) hinausragt und optional eine Antennenfläche von 30 mm² bis 100 mm² aufweist und optional dazu ausgelegt ist, in einem in ein Auge implantierten Zustand des ophthalmischen Implantats (100) den Kapselsack des Auges aufzuspannen.

4. Ophthalmisches Implantat (100) gemäß Anspruch 2 oder 3, wobei die Antennenstruktur (112a) eine oder mehrere Leiterbahnen (114) aufweist, welche
- zumindest teilweise in geradlinig verlaufenden parallelen Windungen verlaufen; und/oder
- zumindest teilweise mäanderförmig verlaufen.

5. Ophthalmisches Implantat (100) gemäß einem der Ansprüche 2 bis 4, wobei der nicht-optische Teil (110) zumindest eine armförmige Haptik aufweist und wobei die Antennenstruktur (112b) zumindest teilweise in einem Bereich der zumindest einen armförmigen Haptik angeordnet ist, der bei einer gebrauchsüblichen Verformung der armförmigen Haptik die geringsten mechanischen Spannungen aufweist.

6. Ophthalmisches Implantat (100) gemäß einem der Ansprüche 2 bis 5, wobei die Antennenstruktur (112b) derart ausgebildet ist, dass diese in einem in ein Auge implantierten Zustand des ophthalmischen Implantats (100) zumindest teilweise den Kapselsack des Auges aufspannt, und wobei die Antennenstruktur (112b) optional derart ausgebildet ist, dass in implantiertem Zustand des ophthalmischen Implantats (100) die Antennenstruktur eine periodische Strukturierung der Antennenoberfläche und/oder Kanten aufweist.

7. Ophthalmisches Implantat (100) gemäß einem der Ansprüche 2 bis 6, wobei die Antennenstruktur (112b) derart ausgebildet ist, dass diese den optischen Teil (108) und/oder den nicht-optischen Teil (110), insbesondere eine Haptik des nicht-optischen Teils, mechanisch verstärkt und/oder stabilisiert.

8. Ophthalmisches Implantat (100) gemäß einem der vorhergehenden Ansprüche, wobei das ophthalmische Implantat (100) als Intraokularlinse (102) ausgebildet ist und/oder wobei der optische Teil (108) eine Linse mit einer refraktiven und/oder diffraktiven Wirkung aufweist oder als solche ausgebildet ist.

9. Ophthalmisches Implantat (100) gemäß einem der vorhergehenden Ansprüche, wobei der nicht-optische Teil (110) eine oder mehrere Haptiken aufweist.

10. Ophthalmisches Implantat (100) gemäß einem der vorhergehenden Ansprüche, wobei die elektronisch auslesbaren Daten eine oder mehrere der folgenden Informationen umfassen:
- eine eindeutige Identifikationsbezeichnung zur Identifikation des ophthalmischen Implantats (100);
- eine Information über eine oder mehrere Eigenschaften des Implantats (100), insbesondere eine optische Eigenschaft, eine Materialeigenschaft, eine Eigenschaft betreffend eine Haptik-Plattform, eine Eigenschaft betreffend ein IOL-Design, und/oder eine Eigenschaft betreffend einen Herstellungsprozess des ophthalmischen Implantats (100);
- eine Information über den Patienten, in welchen das ophthalmische Implantat (100) implantiert ist oder für welchen das ophthalmische Implantat (100) vorgesehen ist;
- eine Information über die Implantation des ophthalmischen Implantats (100);
- eine von einem mit dem ophthalmischen Implantat (100) verbundenen Sensor bereitgestellte Information;
- eine Netzwerkadresse, an welcher Daten mit Informationen über das ophthalmische Implantat (100) und/oder den Patienten hinterlegt und/oder hinterlegbar sind.

11. Ophthalmisches Implantat (100) gemäß einem der vorhergehenden Ansprüche, wobei die kontaktlos elektronisch auslesbare Datenspeichereinheit (112) mittels zumindest einer der folgenden Befestigungsarten auf den nicht-optischen (110) und/oder den optischen Teil (108) aufgebracht ist: Verkleben, Laminieren

12. Ophthalmisches Implantat (100) gemäß einem der vorhergehenden Ansprüche, wobei die kontaktlos elektronisch auslesbare Datenspeichereinheit (112) ein RFID-Tag umfasst oder als solches ausgebildet ist.

13. Ophthalmisches Implantat (100) gemäß einem der vorhergehenden Ansprüche, wobei die kontaktlos elektronisch auslesbare Datenspeichereinheit (112) mittels eines separaten Lesegerätes kontaktlos auslesbar ist, wobei ein Abstand der kontaktlos elektronisch auslesbaren Datenspeichereinheit zum Lesegerät während eines Auslesevorgangs optional im Bereich 5 mm bis 300 mm, optional 10 mm bis 200 mm, optional 25 mm bis 75 mm wählbar ist; und/oder wobei die kontaktlos elektronisch auslesbare Datenspeichereinheit (112) dazu ausgelegt ist, elektronische Signale zu empfangen und/oder zu senden, welche in einem bestimmten Frequenzbereich zwischen 100 kHz und 6 GHz liegen.

14. Ophthalmisches Implantat (100) gemäß einem der vorhergehenden Ansprüche, wobei der optische Teil (108) und/oder der nicht-optische Teil (110) jeweils aus mehreren Elementen ausgebildet sind.

15. Verfahren zur Herstellung eines ophthalmischen Implantats (100), das Verfahren umfassend die folgenden Schritte:
- Bereitstellen eines ophthalmischen Implantats (100) mit einem optischen Teil (108) und einem mit dem optischen Teil (108) verbundenen oder verbindbaren nicht-optischen Teil (110);
- Aufbringen einer kontaktlos elektronisch auslesbaren Datenspeichereinheit (112) derart, dass die optische Wirkung des optischen Teils im Wesentlichen nicht durch die elektronisch auslesbare Datenspeichereinheit (112) beeinträchtigt wird.
**dadurch gekennzeichnet, dass**
die kontaktlos elektronisch auslesbaren Datenspeichereinheit (112) derart ausgebildet ist, dass ein Elastizitätsmodul und/oder ein Wärmeausdehnungskoeffizient der kontaktlos elektronisch auslesbaren Datenspeichereinheit (112) im Wesentlichen einem Elastizitätsmodul und/oder einem Wärmeausdehnungskoeffizienten des optischen Teils (108) und/oder des nicht-optischen Teils (110) entspricht.

## Claims

1. Ophthalmic implant (100), comprising:
- an optical part (108) with an optical effect,
- a non-optical part (110) connected or connectable to the optical part (108),
- a data storage unit (112) which can be read out electronically in a contactless manner when the ophthalmic implant (100) is in the implanted state and which contains data which can be read out electronically in a contactless manner, said data rendering the ophthalmic implant (100) identifiable;
wherein the data storage unit (112) which can be read out electronically is applied to the non-optical (110) and/or the optical part (108) and is arranged in such a way that the optical effect of the optical part (108) is substantially not impaired by the data storage unit (112) which can be read out electronically,
**characterized in that**
a Young's modulus and/or a coefficient of thermal expansion of the data storage unit (112) which can be read out electronically in a contactless manner substantially corresponds to a Young's modulus and/or a coefficient of thermal expansion of the optical part (108) and/or the non-optical part (110).

2. Ophthalmic implant (100) according to Claim 1, wherein the data storage unit (112) which can be read out electronically in a contactless manner comprises a data storage element and an antenna structure.

3. Ophthalmic implant (100) according to Claim 2, wherein the antenna structure (112b)
- is arranged exclusively on a haptic of the non-optical part (110) and optionally has an antenna area of 2 mm² to 5 mm²; or
- is arranged on one or more haptics of the non-optical part (110) and in a peripheral region of the optical part (110) and optionally has an antenna area of 6 mm² to 30 mm²; or
- is arranged on the ophthalmic implant (100) in such a way that the antenna structure (112b) extends along an outer contour of the ophthalmic implant (100) and optionally has an antenna area of 6 mm² to 30 mm²; or
- is arranged in such a way that the antenna structure (112b) extends beyond the outer contour of the optical part (108) and the non-optical part (110) of the ophthalmic implant (100) and optionally has an antenna area of 30 mm² to 100 mm² and is optionally designed to span the capsular bag of the eye when the ophthalmic implant (100) is in an implanted state in an eye.

4. Ophthalmic implant (100) according to Claim 2 or 3, wherein the antenna structure (112a) comprises one or more conductor paths (114), which
- extend, at least in part, in straight, mutually parallel turns; and/or
- extend, at least in part, in meandering fashion.

5. Ophthalmic implant (100) according to any of Claims 2 to 4, wherein the non-optical part (110) comprises at least one arm-shaped haptic and wherein the antenna structure (112b) is arranged, at least in part, in a region of the at least one arm-shaped haptic which has the lowest mechanical stresses in a customary deformation of the arm-shaped haptic during use.

6. Ophthalmic implant (100) according to any of Claims 2 to 5, wherein the antenna structure (112b) is designed such that it at least partially spans the capsular bag of the eye when the ophthalmic implant (100) is in an implanted state in an eye, and wherein the antenna structure (112b) is optionally designed such that the antenna structure has a periodic structuring of the antenna surface and/or edges when the ophthalmic implant (100) is in the implanted state.

7. Ophthalmic implant (100) according to any of Claims 2 to 6, wherein the antenna structure (112b) is designed such that it mechanically strengthens and/or stabilizes the optical part (108) and/or the non-optical part (110), in particular a haptic of the non-optical part.

8. Ophthalmic implant (100) according to any of the preceding claims, wherein the ophthalmic implant (100) is designed as an intraocular lens (102) and/or wherein the optical part (108) comprises a lens with a refractive and/or diffractive effect or is designed as such.

9. Ophthalmic implant (100) according to any of the preceding claims, wherein the non-optical part (110) comprises one or more haptics.

10. Ophthalmic implant (100) according to any of the preceding claims, wherein the data which can be read out electronically comprise one or more of the following pieces of information:
- a unique identifier for identifying the ophthalmic implant (100);
- a piece of information about one or more properties of the implant (100), in particular an optical property, a material property, a property relating to a haptic platform, a property relating to an IOL design, and/or a property relating to a manufacturing process for the ophthalmic implant (100);
- a piece of information about the patient in whom the ophthalmic implant (100) has been implanted or for whom the ophthalmic implant (100) is intended;
- a piece of information regarding the implantation of the ophthalmic implant (100);
- a piece of information provided by a sensor connected to the ophthalmic implant (100);
- a network address where data with information about the ophthalmic implant (100) and/or the patient are stored and/or can be stored.

11. Ophthalmic implant (100) according to any of the preceding claims, wherein the data storage unit (112) which can be read out electronically in a contactless manner is applied to the non-optical (110) and/or the optical part (108) by means of at least one of the following attachment types: adhesive bonding, laminating.

12. Ophthalmic implant (100) according to any of the preceding claims, wherein the data storage unit (112) which can be read out electronically in a contactless manner comprises an RFID tag or is designed as such.

13. Ophthalmic implant (100) according to any of the preceding claims, wherein the data storage unit (112) which can be read out electronically in a contactless manner can be read out in a contactless manner by means of a separate reader, wherein a distance during a read-out process between the data storage unit which can be read out electronically in a contactless manner and the reader can be optionally chosen in the range from 5 mm to 300 mm, optionally from 10 mm to 200 mm, optionally 25 mm to 75 mm; and/or wherein the data storage unit (112) which can be read out electronically in a contactless manner is designed to receive and/or transmit electronic signals which are located in a certain frequency range between 100 kHz and 6 GHz.

14. Ophthalmic implant (100) according to any of the preceding claims, wherein the optical part (108) and/or the non-optical part (110) are each formed of multiple elements.

15. Method for manufacturing an ophthalmic implant (100), the method comprising the following steps:
- providing an ophthalmic implant (100) having an optical part (108) and a non-optical part (110) connected or connectable to the optical part (108);
- applying a data storage unit (112) which can be read out electronically in a contactless manner, the application being such that the optical effect of the optical part is substantially not impaired by the data storage unit (112) which can be read out electronically,
**characterized in that**
the data storage unit (112) which can be read out electronically in a contactless manner is designed such that a Young's modulus and/or a coefficient of thermal expansion of the data storage unit (112) which can be read out electronically in a contactless manner substantially corresponds to a Young's modulus and/or a coefficient of thermal expansion of the optical part (108) and/or the non-optical part (110).

## Revendications

1. Implant ophtalmique (100), comprenant :
- une partie optique (108) ayant un effet optique ;
- une partie non optique (110) reliée ou pouvant être reliée à la partie optique (108) ;
- une unité de stockage de données (112) qui est lisible électroniquement sans contact à l'état implanté de l'implant ophtalmique (100) et qui contient des données lisibles électroniquement sans contact, sur la base desquelles l'implant ophtalmique (100) est identifiable ;
l'unité de stockage de données lisibles électroniquement (112) étant appliquée à la partie non optique (110) et/ou à la partie optique (108) et étant disposée de telle sorte que l'effet optique de la partie optique (108) soit sensiblement non altéré par l'unité de stockage de données lisibles électroniquement (112).
**caractérisé en ce que**
un module d'élasticité et/ou un coefficient de dilatation thermique de l'unité de stockage de données lisibles électroniquement sans contact (112) correspond sensiblement à un module d'élasticité et/ou à un coefficient de dilatation thermique de la partie optique (108) et/ou de la partie non optique (110).

2. Implant ophtalmique (100) selon la revendication 1, dans lequel l'unité de stockage de données lisibles électroniquement sans contact (112) présente un élément de stockage de données et une structure d'antenne.

3. Implant ophtalmique (100) selon la revendication 2, dans lequel la structure d'antenne (112b)
- est disposée exclusivement sur une haptique de la partie non optique (110) et présente facultativement une surface d'antenne de 2 mm² à 5 mm² ; ou
- est disposée sur une ou plusieurs haptiques de la partie non optique (110) et dans une région périphérique de la partie optique (110) et présente facultativement une surface d'antenne de 6 mm² à 30 mm² ; ou
- est disposée sur l'implant ophtalmique (100) de telle sorte que la structure d'antenne (112b) s'étende le long d'un contour extérieur de l'implant ophtalmique (100) et présente facultativement une surface d'antenne de 6 mm² à 30 mm² ; ou
- est disposée de telle sorte que la structure d'antenne (112b) dépasse du contour extérieur de la partie optique (108) et de la partie non optique (110) de l'implant ophtalmique (100) et présente facultativement une surface d'antenne de 30 mm² à 100 mm² et est facultativement configurée pour sous-tendre le sac capsulaire de l'œil dans un état implanté dans un œil de l'implant ophtalmique (100).

4. Implant ophtalmique (100) selon la revendication 2 ou 3, dans lequel la structure d'antenne (112a) présente une ou plusieurs pistes conductrices (114), lesquelles
- s'étendent au moins partiellement en spires parallèles rectilignes ; et/ou
- s'étendent au moins partiellement en forme de méandres.

5. Implant ophtalmique (100) selon l'une quelconque des revendications 2 à 4, dans lequel la partie non optique (110) présente au moins une haptique en forme de bras et dans lequel la structure d'antenne (112b) est disposée au moins partiellement dans une zone de l'au moins une haptique en forme de bras qui présente les contraintes mécaniques les plus faibles lors d'une déformation habituelle de l'haptique en forme de bras.

6. Implant ophtalmique (100) selon l'une quelconque des revendications 2 à 5, dans lequel la structure d'antenne (112b) est réalisée de telle sorte que, dans un état implanté dans un œil de l'implant ophtalmique (100), elle recouvre au moins partiellement le sac capsulaire de l'œil, et dans lequel la structure d'antenne (112b) est facultativement réalisée de telle sorte que, à l'état implanté de l'implant ophtalmique (100), la structure d'antenne présente une structuration périodique de la surface d'antenne et/ou des bords.

7. Implant ophtalmique (100) selon l'une quelconque des revendications 2 à 6, **caractérisé en ce que** la structure d'antenne (112b) est réalisée de telle sorte qu'elle renforce et/ou stabilise mécaniquement la partie optique (108) et/ou la partie non optique (110), en particulier une haptique de la partie non optique.

8. Implant ophtalmique (100) selon l'une quelconque des revendications précédentes, dans lequel l'implant ophtalmique (100) est réalisé sous la forme d'une lentille intraoculaire (102) et/ou dans lequel la partie optique (108) présente ou est réalisée sous la forme d'une lentille à effet réfractif et/ou diffractif.

9. Implant ophtalmique (100) selon l'une quelconque des revendications précédentes, dans lequel la partie non optique (110) comporte une ou plusieurs haptiques.

10. Implant ophtalmique (100) selon l'une quelconque des revendications précédentes, dans lequel les données lisibles électroniquement comprennent une ou plusieurs des informations suivantes :
- une désignation d'identification unique pour l'identification de l'implant ophtalmique (100) ;
- une information relative à une ou plusieurs propriétés de l'implant (100), notamment une propriété optique, une propriété matérielle, une propriété relative à une plate-forme haptique, une propriété relative à une conception de IOL et/ou une propriété relative à un processus de production de l'implant ophtalmique (100) ;
- une information relative au patient chez lequel l'implant ophtalmique (100) est implanté ou pour lequel l'implant ophtalmique (100) est prévu ;
- une information relative à l'implantation de l'implant ophtalmique (100) ;
- une information fournie par un capteur relié à l'implant ophtalmique (100) ;
- une adresse réseau à laquelle sont et/ou peuvent être stockées des données contenant des informations relatives à l'implant ophtalmique (100) et/ou au patient.

11. Implant ophtalmique (100) selon l'une quelconque des revendications précédentes, dans lequel l'unité de stockage de données (112) lisible électroniquement sans contact est appliquée à la partie non optique (110) et/ou à la partie optique (108) au moyen d'au moins l'un des types de fixation suivants : collage, stratification.

12. Implant ophtalmique (100) selon l'une quelconque des revendications précédentes, dans lequel l'unité de stockage de données (112) lisible électroniquement sans contact comprend ou est réalisée sous la forme d'une étiquette RFID.

13. Implant ophtalmique (100) selon l'une quelconque des revendications précédentes, dans lequel l'unité de stockage de données lisibles électroniquement sans contact (112) est lisible sans contact au moyen d'un dispositif de lecture séparé, dans lequel une distance de l'unité de stockage de données lisibles électroniquement sans contact au dispositif de lecture pendant une opération de lecture est facultativement de l'ordre de 5 mm à 300 mm, facultativement de 10 mm à 200 mm, facultativement de 25 mm à 75 mm ; et/ou dans lequel l'unité de stockage de données lisibles électroniquement sans contact (112) est configurée pour recevoir et/ou émettre des signaux électroniques qui se situent dans une plage de fréquences déterminée comprise entre 100 kHz et 6 GHz.

14. Implant ophtalmique (100) selon l'une quelconque des revendications précédentes, dans lequel la partie optique (108) et/ou la partie non optique (110) sont respectivement formées d'une pluralité d'éléments.

15. Procédé de fabrication d'un implant ophtalmique (100), le procédé comprenant les étapes suivantes :
- fournir un implant ophtalmique (100) présentant une partie optique (108) et une partie non optique (110) reliée ou ou pouvant être reliée à la partie optique (108) ;
- application d'une unité de stockage de données (112) lisible électroniquement sans contact de telle sorte que l'effet optique de la partie optique ne soit sensiblement pas altéré par l'unité de stockage de données (112) lisible électroniquement.
**caractérisé en ce que**
l'unité de stockage de données (112) lisible électroniquement sans contact est conçue de telle sorte qu'un module d'élasticité et/ou un coefficient de dilatation thermique de l'unité de stockage de données (112) lisible électroniquement sans contact corresponde sensiblement à un module d'élasticité et/ou à un coefficient de dilatation thermique de la partie optique (108) et/ou de la partie non optique (110).
